**Europäisches Patentamt**

**European Patent Office**

**Office européen des brevets**

(11) Numéro de publication: **0 098 332**
A1

## (12) DEMANDE DE BREVET EUROPEEN

(21) Numéro de dépôt: 82200830.6

(22) Date de dépôt: 02.07.82

(51) · Int. Cl.³: **C 12 P 7/06**, C 12 P 7/16,
C 12 F 1/00

(43) Date de publication de la demande: 18.01.84
Bulletin 84/3

(71) Demandeur: **ATELIERS DE CONSTRUCTIONS ELECTRIQUES DE CHARLEROI (ACEC) Société Anonyme, Avenue Lloyd George 7, B-1050 Bruxelles (BE)**

(72) Inventeur: **Soudan, née Molnet, Claire, 10 Drève des Equipages, B-1170 Bruxelles (BE)**
Inventeur: **Rongy, André, Avenue G.E. Lebon, 25, B-1160 Bruxelles (BE)**
Inventeur: **Bingen, Roald, 41, Avenue des Genêts, B-6001 Marcinelle (BE)**

(84) Etats contractants désignés: **AT BE CH DE FR GB IT LI LU NL SE**

(74) Mandataire: **Bossard, Franz et al, ACEC - Service des Brevets Boîte Postale 4, B-6000 Charleroi (BE)**

(54) Procédé et installation de fermentation et de distillation en continu.

(57) Dans un procédé de production de fluides organiques par distillation à partir d'un substrat en milieu aqueux, soumis à une fermentation continue dans un fermenteur (2) dont l'effluent est amené dans un évaporateur (11) où il est soumis à une évaporation sous pression et température réduites, les vapeurs de liquide organique sortant de l'évaporateur (11) sont comprimées dans un compresseur (13) disposé immédiatement à la sortie de l'évaporateur (11).

EP 0 098 332 A1

1

PROCEDE ET INSTALLATION DE FERMENTATION ET DE DISTILLATION EN CONTINU.

La présente invention a pour objet un procédé et une installation de fermentation par levures, bactéries ou champignons d'un substrat composé de substances organiques en solution ou en émulsion dans de l'eau et de distillation en continu du produit de la fermentation d'une façon particulièrement économique du point de vue de la consommation d'énergie. Ce procédé peut par exemple s'appliquer à la fabrication d'alcools tels que l'alcool éthylique, méthylique, butylique, propylique, le glycérol, le furfurol, de tryptophane de cétones ou encore de toute substance organique liquide aux températures normales d'utilisation, produite par fermentation en milieu aqueux et séparable de l'eau par distillation.

On connaît déjà des procédés de production de liquides organiques par distillation à partir d'un substrat en milieu aqueux soumis à une fermentation continue dans un fermenteur dont l'effluent est amené dans un évaporateur où il est soumis à une évaporation sous pression et température réduites et dans lequel on obtient la pression réduite dans l'évaporateur en reliant ce dernier à l'entrée d'un compresseur. Dans ces procédés connus, la distillation ultérieure est réalisée aussi sous pression réduite, ce qui nécessite des colonnes de distillation de très grand volume et par conséquence très coûteuses.

Le procédé selon la présente invention n'a pas cet inconvénient, du fait que le compresseur est en amont de la colonne de distillation, et que par conséquent celle-ci peut fonctionner à une pression voisine de la pression atmosphérique, ce qui en diminue considérablement les dimensions et le coût.

Suivant l'invention, les vapeurs de liquide organique sortant de l'évaporateur sont comprimées immédiatement dans le compresseur dont le fonctionnement est réglé pour obtenir à la sortie et après refroidissement, une vapeur à une pression voisine de la pression de l'air ambiant et à une température supérieure mais voisine à la température de condensation de ces vapeurs. De plus, des échangeurs de chaleur récupérant la chaleur dans des circuits de refroidissement des vapeurs sortant du compresseur alimentent d'autres échangeurs de chaleur permettant de chauffer l'évaporateur et éventuellement un circuit de reflux de liquide

20.18/1877 .

appauvri en distillat au bas d'une colonne de distillation.

L'invention est décrite ci-dessous par rapport à un exemple d'une forme d'exécution en se référant aux dessins annexés. Les deux figures du dessin représentent des schémas de fonctionnement d'une installation de production d'éthanol par fermentation à l'aide de levures à partir d'un substrat constitué d'une solution de glucose dans de l'eau.

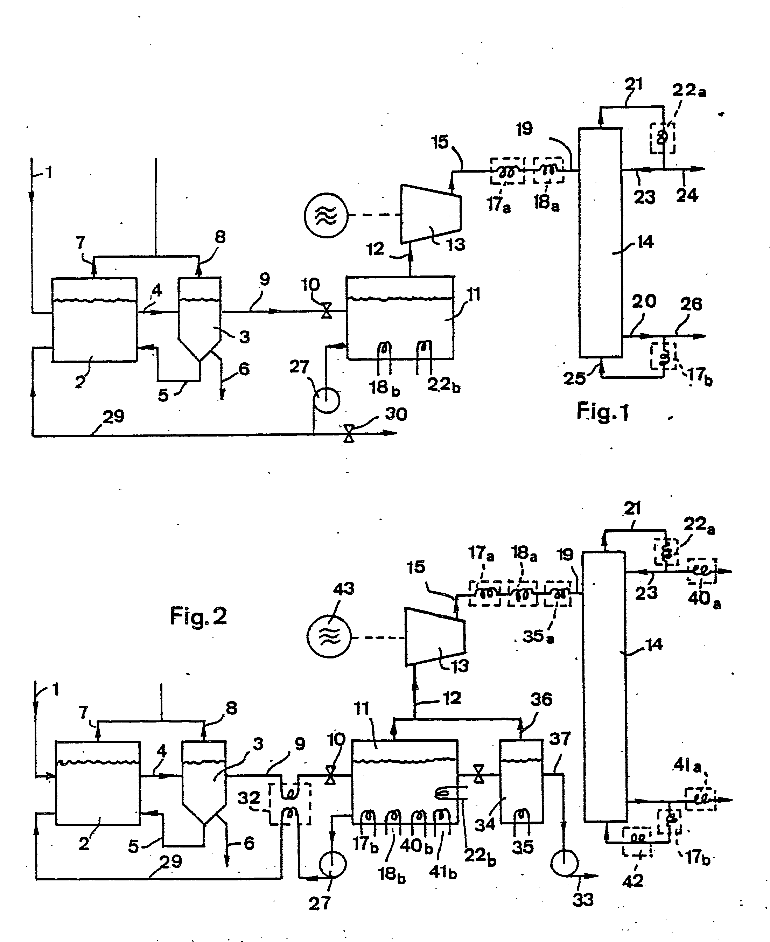

La figure 1 donne le schéma de principe d'une installation dans laquelle on réalise le procédé selon l'invention.

Une solution aqueuse de glucose d'un titre pondéral compris par exemple entre 5 et 50%, est introduite par un conduit d'alimentation 1 dans une installation de fermentation en continu composé d'un fermenteur 2 et d'un séparateur 3. Dans le fermenteur 2, on maintient une température $t_o$, favorable pour le métabolisme des levures, de l'ordre de 35°C. Cette température $t_o$ y est maintenue de préférence grâce à la chaleur dégagée lors de la fermentation. Le liquide alcoolisé produit par la fermentation sort du fermenteur 2 par un conduit 4 et entre dans le séparateur 3 où les levures sont séparées du liquide alcoolisé par sédimentation et/ou centrifugation. Ce séparateur a pour but d'éviter la perte d'une partie des levures et d'éviter aussi une fermentation dans les parties de l'installation en aval. Dans ce séparateur 3, les levures sont piégées et recyclées vers le fermenteur 1 via un conduit 5. Une purge 6 permettant l'évacuation des levures de faible rendement peut être prévue. Le gaz d'acide carbonique anhydre formé pendant la fermentation est évacué via un conduit 7 sortant du fermenteur 2 et éventuellement un conduit 8 sortant du séparateur 3. Le liquide alcoolisé sortant du séparateur 3 par un conduit 9 entre ensuite via un détendeur 10 dans un évaporateur 11, relié au moyen d'un conduit de grand diamètre 12 à l'entrée d'un compresseur 13, qui maintient dans l'évaporateur 1 une pression $p_1$, basse par rapport à la pression atmosphérique. Cette pression $p_1$ est la pression d'équilibre entre la phase liquide et la phase vapeur pour laquelle il y a ébullition du mélange liquide eau-alcool-glucose dans l'évaporateur 11, à une température $t_1$ régnant dans ce dernier. Cette température $t_1$ est voisine de la température $t_o$ qui règne dans le fermenteur 2, soit par exemple environ 35°C, dans quel cas la pression d'équilibre $p_1$ régnant dans l'évaporateur 11 est de l'ordre de 70 mbar absolus environ.

Les vapeurs d'eau et d'éthanol produites par l'ébullition du liquide alcoolisé dans l'évaporateur 11 sont comprimées à l'aide du compresseur 13 jusqu'à une pression $p_2$ légèrement supérieure à la pression $p_3$ régnant dans une colonne de distillation fractionnée 14; les vapeurs y atteignent une température $t_2$. Elles sont ensuite envoyées par un conduit 15 dans un ou plusieurs échangeurs de chaleur $17_a$ et $18_a$ puis par un conduit 19 dans la colonne de distillation fractionnée 14, à une température $t_3$ comprise entre la température $t_4$ d'ébullition du résidu de distillation riche en eau, véhiculé dans un conduit 20 et la température $t_5$ d'ébullition du distillat riche en éthanol, véhiculé dans un conduit 21 à la pression $p_3$ régnant dans la colonne de distillation. Cette dernière peut avantageusement être pourvue d'un reflux des produits de tête, selon la technique connue consistant à prélever les produits de tête par le conduit 21, les condenser dans un échangeur $22_a$ et renvoyer le reflux par un conduit 23, le produit à forte teneur en éthanol sortant par un conduit 24. De la même façon, au pied de la colonne de distillation, une partie du résidu de distillation riche en eau, recueillie dans le conduit 20, est recyclée par un conduit 25 et selon la technique connue vaporisée dans un échangeur $17_b$, tandis que le solde est évacué par un conduit 26.

La chaleur nécessire pour vaporiser l'eau et l'éthnaol dans l'évaporateur 11 est fournie de préférence par une partie des vapeurs chaudes sortant du compresseur 13, grâce à un échangeur de chaleur qui, pour la clarté du dessin, figure sous forme de deux unités, une première $18_a$ entre le compresseur 13 et la colonne de distillation 14 et une deuxième $18_b$ dans l'évaporateur 11. Cette chaleur de vaporisation peut être fournie aussi en partie par la récupération de la chaleur de condensation du produit de tête de la colonne de distillation 14 grâce à un échangeur qui de façon similaire est représenté sous forme de deux unités $22_a$ et $22_b$.

En outre, la chaleur nécessaire pour bouillir le reflux riche en eau réinjecté par le conduit 25 à la base de la colonne de distillation 14 peut être fournie soit partiellement soit totalement par les vapeurs chaudes sortant du compresseur 13, grâce à l'échangeur de chaleur dont l'unité $17_a$ représente le circuit primaire et l'unité $17_b$ le circuit secondaire.

Selon l'invention, les pressions $p_1$ dans l'évaporateur 1 et $p_3$ dans la colonne de distillation 14 doivent être choisies de telle façon que la température correspondante $t_1$ du liquide alcoolisé dans l'évaporateur 11 soit inférieure à la température $t_5$ de la vapeur riche en éthanol dans le conduit 21 en tête de la colonne de distillation 14, et que la température $t_2$ des gaz à la sortie du compresseur 13 soit supérieure à la température $t_4$ d'ébullition de l'eau.

Afin de réduire le coût de la colonne de distillation, il est avantageux d'adopter une pression $p_3$ voisine de la pression atmosphérique. Dans ce cas, et pour $p_1$ voisin de 70 mbar et $t_1$ voisine de 35°C, les températures $t_2$, $t_3$, $t_4$ et $t_5$ sont voisines respectivement de 260°C, 93°C, 99°C et 78°C ce qui permet effectivement les récupérations de chaleurs décrites ci-dessus.

L'évaporateur 11 pourra avantageusement être constitué de plusieurs sections non représentées sur la figure 1 parcourues à la suite l'une de l'autre par le liquide alcoolisé, de façon à ne pas mélanger le liquide appauvri en éthanol à la sortie avec le liquide riche en éthanol à l'entrée de l'évaporateur 11. Une pompe 27 permet d'extraire le liquide appauvri en éthanol en le ramenant à la pression atmosphérique et en l'évacuant par une purge 28. Il est avantageux, lorsque le liquide évacué en 28 par la purge a encore une teneur significative en éthanol, d'en recycler la majeure partie par un conduit 29 vers l'entrée du fermenteur 2, le taux de recyclage étant réglé à l'aide d'une vanne 30.

La figure 2 représente une variante de l'installation selon l'invention, différant de celle de la figure 1 décrite ci-dessus par les points suivants :

a) l'évaporateur 11 est à une température $t_1$ plus élevée que la température $t_o$ du fermenteur, tout en restant inférieure à la température $t_5$ du distillat dans la conduite de tête 21 de la colonne de distillation, par exemple $t_1$ de 50 à 70°C pour $t_o$ = 35°C et $t_5$ = 78°C. Cette température $t_1$ peut être réglée en dosant correctement le transfert de chaleur des échangeurs chauffant l'évaporateur 11. Un échangeur supplémentaire 32 permet de récupérer les calories du liquide appauvri circulant dans le conduit de recyclage 29 vers le liquide riche en éthanol circulant dans le conduit 9.

b) La portion du liquide appauvri sortant de l'évaporateur 11, non recyclé vers le fermenteur 2 et évacué par la purge 28, est une nouvelle fois appauvri en éthanol avant élimination par une purge 33 grâce à un évaporateur auxiliaire 34 chauffé par un échangeur $35_b$ puisant sa chaleur par exemple en $35_a$ entre la sortie du compresseur 13 et la colonne de distillation 14 (il pourrait aussi la puiser dans la condensation en distillat en $22_a$). Les vapeurs d'eau et d'alcool sont collectées via un conduit 36 par le compresseur 12, tandis que le liquide restant est évacué via un conduit 37 par une pompe 38 vers la purge 33.

c) les chaleurs sensibles du distillat dans le conduit 24 et du résidu de distillation dans le conduit 26 sont récupérées respectivement par des échangeurs de chaleur $40_a$ et $41_a$ et renvoyées vers l'évaporateur, ainsi qu'il est schématiquement représenté en $40_b$ et $41_b$.

d) Un échangeur de chaleur supplémentaire 42 permet d'introduire dans le système des calories supplémentaires dans le cas où le travail de compression développé par le compresseur 13 et fourni par son moteur d'entraînement 43 ne suffisait pas à maintenir les différentes températures à un niveau adéquat.

Aussi bien dans le cas de la figure 1 que dans le cas de la figure 2, il est possible de faire fonctionner l'installation sans autre apport d'énergie que celle dégagée lors de la fermentation et celle fournie par le moteur 43 entraînant le compresseur 13. De plus, sauf les évaporateurs 11 et 34 et les conduits 12 et 36 à l'entrée du compresseur 13, aucune autre partie de l'installation n'est sous vide et ne nécessite des sections de passage outre mesure larges, ni des joints d'étanchéité résistants à des pressions élevées.

REVENDICATIONS

1. Procédé de production de liquides organiques par distillation à partir d'un substrat en milieu aqueux soumis à une fermentation continue dans une fermenteur (2) dont l'effluent est amené dans un évaporateur (11) où il est soumis à une évaporation sous pression et température réduites,

caractérisé en ce que les vapeurs d'eau et de liquide organique sortant de l'évaporateur (11) sont comprimées dans un compresseur (13) disposé immédiatement à la sortie de l'évaporateur (11).

2. Procédé suivant la revendication 1, caractérisé en ce que le fonctionnement du compresseur (13) est réglé pour obtenir à sa sortie et après refroidissement une vapeur à une pression voisine à la pression de l'air ambiant et à une température voisine, légèrement supérieure à sa température de condensation.

3. Procédé suivant une des revendications 1 ou 2, caractérisé en ce que le refroidissement de la vapeur sortant du compresseur (13) est réalisé dans au moins un échangeur de chaleur ($17_a$, $17_b$; $18_a$, $18_b$; $35_a$, $35_b$) permettant d'utiliser la chaleur récupérée pour chauffer l'évaporateur.

4. Procédé suivant une quelconque des revendications précédentes, caractérisé en ce que la vapeur sortant du compresseur puis refroidie à une température voisine mais supérieure à sa température de condensation est introduite dans au moins une colonne de distillation (14) et/ou de rectification.

5. Procédé suivant une quelconque des revendications précédentes, caractérisé en ce que le rapport de compression du compresseur (13) est réglé de telle façon que la température d'ébullition du mélange liquide dans l'évaporateur, à la pression réduite engendrée par le compresseur, soit sensiblement inférieure à la température de condensation du produit de tête dans la colonne de distillation et/ou de rectification.

6. Procédé suivant la revendication 4 ou 5, caractérisé en ce que les produits de tête et de queue de ces colonnes de distillation et/ou de rectification sont repris dans des circuits de reflux équipés d'échangeurs de chaleur ($17_b$, $22_a$) associés aux échangeurs de chaleur ($22_b$, $17_a$) dans l'évaporateur (11) et à la sortie du compresseur (13).

20.18/1877.

7.   Procédé suivant une quelconque des revendications précédentes, caractérisé en ce que l'évaporateur (11) est doublé d'un évaporateur auxilaire (34) dont le résidu est évacué par une purge (33).

8.   Procédé suivant une quelconque des revendications précédentes, caractérisé en ce que l'évaporateur est à une température supérieure à celle du fermenteur et, qu'on récupère la chaleur du circuit de recyclage de l'évaporateur (11) vers le fermenteur (2) au moyen d'un échangeur (32) entre le circuit de recyclage et le circuit d'alimentation de l'évaporateur (11).

1\1  0098332

Fig.1

Fig.2

Office européen
des brevets

**RAPPORT DE RECHERCHE EUROPEENNE**

0098332
Numéro de la demande

EP 82 20 0830

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | C 12 P 7/06 |
| X | EP-A-0 011 334 (PROCESS ENGINEERING CO. S.A.) * revendications 1,2,5,6,7,9; figure 1; page 6, ligne 21 - page 7, ligne 5 * | 1,2,3, 4 | C 12 P 7/16 C 12 F 1/00 |
| | --- | | |
| X | GB-A-2 054 643 (KINS DEVELOPMENTS LTD) * revendications 1,2,3; page 4, lignes 50-53; figure 1 * | 1,2,3 | |
| | --- | | |
| X | WO-A-8 103 182 (DELAIR C.M.) * revendications 1,2,3; figure 1; page 8, lignes 12-24 * | 1,2,3 | |
| | ----- | | |

**DOMAINES TECHNIQUES RECHERCHES (Int. Cl. ³)**

C 12 P
C 12 F
B 01 D

Le présent rapport de recherche a été établi pour toutes les revendications

| Lieu de la recherche | Date d'achèvement de la recherche | Examinateur |
|---|---|---|
| LA HAYE | 18-01-1983 | ALMOND C.A. |